# EUROPÄISCHE PATENTSCHRIFT

(11) **EP 0 716 080 B1**
(45) Veröffentlichungstag und Bekanntmachung des Hinweises auf die Patenterteilung: **26.08.1998**
(21) Anmeldenummer: 95118903.4
(22) Anmeldetag: 01.12.1995
(51) Int. Cl.: C07C 275/62

(54) **Verfahren zur Herstellung von Biuretgruppen enthaltenden Polyisocyanaten**
Process for the preparation of biuret groups which contain polyisocyanates
Procédé de préparation de polyisocyanates à groupement biuret

(30) Priorität: 09.12.1994 DE 4443885
(43) Veröffentlichungstag der Anmeldung: 12.06.1996
(73) Patentinhaber: BASF Aktiengesellschaft, 67063 Ludwigshafen (DE)
(72) Erfinder: Wolff, Stefan, Dr., D-67117 Limburgerhof (DE); Heider, Wolgang, D-67434 Neustadt (DE); Langer, Werner, Dr., D-67061 Ludwigshafen (DE); Renz, Hans, Dr., D-67149 Meckenheim (DE)

(56) Entgegenhaltungen:
- EP-A- 0 150 769
- EP-A- 0 177 059
- EP-A- 0 183 150
- EP-A- 0 251 952
- EP-A- 0 259 233
- DE-B- 1 174 760
- PATENT ABSTRACTS OF JAPAN vol. 7 no. 201 (C-184) [1346] ,6.September 1983 & JP-A-58 098327 (TAKEDA YAKUHIN KOGYO K.K.) 11.Juni 1983,

## Beschreibung

Gegenstand der Erfindung ist ein Verfahren zur Herstellung von Biuretgruppen enthaltenden Polyisocyanaten durch katalytische Umsetzung von aliphatischen und/oder cycloaliphatischen Diisocyanaten mit einem Biuretisierungsmittel, wobei erfindungsgemäß als Biuretisierungsmittel Wasser in feindisperser Form eingesetzt wird und die Reaktion im Beisein von OH-aciden Verbindungen erfolgt.

Biuretgruppen enthaltende aliphatische und cycloaliphatische Polyisocyanate werden unter anderem in hochwertigen licht- und wetterbeständigen Zweikomponenten-Polyurethanlacken eingesetzt.

Die Herstellung von Polyisocyanaten mit Biuretstruktur ist seit langem bekannt. Sie erfolgt üblicherweise durch Umsetzung von Diisocyanaten mit einem Biuretisierungsmittel, wie beispielsweise Wasser oder wasserabspaltende Substanzen, bei höheren Temperaturen und anschließendem Abtrennen von überschüssigem Monomer durch ein- oder mehrstufige Destillation.

So wird bei Verwendung von wasserabspaltenden Substanzen, wie beispielsweise tert. Butanol, als Biuretisierungsmittel unter Säurekatalyse Wasser in situ erzeugt. Dazu sind jedoch meist hohe Reaktionstemperaturen von mehr als 140°C notwendig, wodurch sich die Reaktionsprodukte gelb färben. Aus anwendungstechnischen Gründen, z.B. für den Einsatz als Klarlack, möchte man jedoch nach Möglichkeit farblose Produkte haben. Des weiteren wird durch die Verwendung eines wasserabspaltenden, in der Regel OH-Gruppen haltigen, Biuretisierungsmittels die Bildung von Nebenprodukten gefördert, die keine Biuretstruktur haben. Diese Nebenprodukte können sich ebenfalls anwendungstechnisch ungünstig auswirken oder die Lagerstabilität des Wertproduktes beeinträchtigen oder verfahrenstechnische Probleme bewirken. So entstehen beispielsweise in dem Verfahren nach EP-A-0 150 769 mit α,α,α-trisubstituierter Essigsäure als Biuretisierungskomponente Carbonsäureanhydride, die zusätzlich zum überschüssigen Diisocyanat destillativ entfernt werden müssen und zu einem erheblichen Anteil an schwer zu trennenden, gemischten Fraktionen aus überschüssigem Diisocyanat und Carbonsäureanhydrid führen. Die im Verfahren nach EP-A-0 183 150 verwendeten Hydroxycarbonsäuren reagieren mit den Ausgangsdiisocyanaten unter Bildung eines Urethan-, Carbonsäureanhydrid- und Biuretgruppen enthaltenden Polyisocyanatgemisches.

Aus diesem Grund wäre die Verwendung von Wasser als Biuretisierungsmittel ideal. Bei Einsatz von Wasser ohne Zusätze ergeben sich jedoch zwei gravierende Nachteile:
1. Die Bildung von unlöslichen Polyharnstoffen in der Reaktion läßt sich in der Regel nicht vermeiden.
2. Die erhaltenen Produkte weisen eine schlechte Lagerstabilität bezüglich Monomerrückspaltung auf, wodurch der kennzeichnungspflichtige Grenzwert von 0,5 % schnell überschritten wird, insbesondere bei Lagerung oberhalb Raumtemperatur.

Es wurde versucht, diese Probleme durch eine modifizierte Fahrweise zu umgehen. In der Patentliteratur sind eine Vielzahl von Varianten zur Herstellung von Polyisocyanaten mit Biuretstruktur, bei denen Wasser als Biuretisierungsmittel eingesetzt wird, genannt.

So wird beispielsweise bereits in US-A-4 028 392 die Umsetzung von Isocyanaten mit Wasser im Beisein hydrophiler organischer Lösungsmittel, wie beispielsweise Trialkylphosphate und Ethylenglycolmonomethylacetat, beschrieben. Nach EP-A-259 233 wird die Reaktion in Gegenwart wenigstens einer Carbonsäure und/oder eines Carbonsäureanhydrids durchgeführt. Außerdem ist die Mitverwendung von Methyl- und/oder Ethylestern der Phosphorsäure sowie von Alkoxyalkylcarboxylaten als Lösungsvermittler vorgesehen. Nachteilig ist hierbei, daß durch die Verwendung eines Lösungsmittels oder Lösungsmittelgemisches in den benötigten Mengen eine geringere Raum-Zeit-Ausbeute an Polyisocyanat erreicht wird als ohne Lösungsmitteleinsatz. Ferner muß eine aufwendigere Destillation mit eventueller Lösungsmittelauftrennung erfolgen.

Gemäß EP-B-251 952 werden organische Polyisocyanate mit Biuretstruktur aus Diisocyanaten und Wasser unter einem Gesamtdruck von mindestens 1,2 bar, bei einem Partialdruck des Kohlendioxids von mindestens 0,2 bar, hergestellt. Diese Fahrweise unter Kohlendi-o-oxid bei erhöhtem Druck erfordert aber spezielle Bedingungen und ist deshalb in der Handhabung sehr aufwendig.

Nach DE-C-29 18 739 wird ein Verfahren zur Herstellung von Polyisocyanaten mit Biuretstruktur durch Umsetzung von Hexamethylendiisocyanat (HDI) mit Wasser beschrieben, in dem man das Wasser in Form von Dämpfen im Gemisch mit Luft bzw. einem Inertgas bei einem Feuchtigkeitsgehalt des Gemisches von 0,1 bis 2,0 kg/kg einem bei 110 bis 130°C gehaltenen Reaktionsgemisch zuführt und die Umsetzung bei einer Temperatur von 150 bis 170°C durchführt. Nachteilig hierbei ist, daß bei dieser Reaktion viele unlösliche Polyharnstoffe entstehen. Dies kann dazu führen, daß sich Anlagenteile, insbesondere Leitungen, zusetzen.

Die Aufgabe der vorliegenden Erfindung bestand darin, ein Verfahren zu entwickeln, das die Probleme der bekannten Verfahren zur Herstellung von Biuretgruppen enthaltenden Isocyanaten löst. Insbesondere sollen die Polyharnstoffbildung zumindest weitgehend vermieden sowie eine gute Lagerstabilität bezüglich der Restmonomerenrückspaltung und möglichst farblose Oligobiurete gewährleistet werden.

Diese Aufgabe wird erfindungsgemäß dadurch gelöst, daß als Biuretisierungsmittel Wasser in feindisperser Form eingesetzt wird und die Reaktion im Beisein von OH-aciden Verbindungen, ausgenommen Carbonsäuren, erfolgt.

Gegenstand der Erfindung ist damit ein Verfahren zur Herstellung von Biuretgruppen enthaltenden Polyisocyanaten durch katalytische Umsetzung von aliphatischen und/oder cycloaliphatischen Diisocyanaten mit einem Biuretisierungsmittel, dadurch gekennzeichnet, daß als Biuretisierungsmittel Wasser in feindisperser Form eingesetzt wird und die Reaktion im Beisein von OH-aciden Verbindungen erfolgt.

Die so hergestellten Biuretgruppen enthaltenden Polyisocyanate sind nahezu farblos und weisen einen niedrigen Restmonomerengehalt auf, der auch bei Lagertemperaturen oberhalb Raumtemperatur nur langsam ansteigt und damit auch unter diesen Bedingungen den Arbeitsschutzanforderungen genügt. Sie werden insbesondere in Ein- oder Zweikomponenten-Polyurethan-Lacken, -Klebstoffen und Polyurethandispersionen eingesetzt.

Als Ausgangsprodukte für die Herstellung der Biuretgruppen enthaltenden Polyisocyanate kommen die an sich bekannten aliphatischen und/oder cycloaliphatischen Diisocyanate allein oder in Mischungen untereinder in Betracht, wie beispielsweise Alkylendiisocyanate mit 4 bis 12 Kohlenstoffatomen im Alkylenrest, wie 1,12-Dodecan-diisocyanat, 2-Ethyl-tetramethylen-diisocyanat-1,4, 2-Methyl-pentamethylen-diisocyanat-1,5, Tetramethylen-diisocyanat-1,4 und Hexamethylen-diisocyanat-1,6 (HDI); cycloaliphatische Diisocyanate, wie Cyclohexan-1,3- und -1,4-diisocyanat sowie beliebige Gemische dieser Isomeren. Vorzugsweise eingesetzt werden 2-Butyl-2-ethyl-pentamethylen-diisocyanat, 2-Methylpentamethylendiisocyanat, Isophorondiisocyanat (IPDI) und HDI. Besonders bevorzugt wird phosgenfrei hergestelltes HDI verwendet.

Als Biuretisierungsmittel findet Wasser in feindisperser Form Verwendung. Die feinteilige Einbringung kann durch den Einsatz bekannter geeigneter Vorrichtungen und Reaktoren mit Rührwerk erfolgen. Zur möglichst feindispersen Einbringung von Wasser in das Diisocyanat wird das Wasser vorzugsweise in Form von Wasserdampf eingesetzt.

Wird der Wasserdampfstrom durch ein Inertgas verdünnt, läßt sich der Prozeß noch besser kontrollieren und steuern. Als Inertgas werden erfindungsgemäß vorzugsweise Stickstoff und/oder Kohlendioxid eingesetzt. Der Wasserdampf kann durch Inertgas so verdünnt werden, daß in dem Gasstrom 10 bis 95 Vol.-% Inertgas vorliegt.

Zur Erreichung der gewünschten Eigenschaften, wie der erforderlichen Lagerstabilität der Produkte, wird die Biuretisierung der Diisocyanate erfindungsgemäß durch die feindisperse Einbringung von Wasser bzw. Wasserdampf in Verbindung mit OH-aciden Verbindungen, ausgenommen Carbonsäuren, durchgeführt. Durch den Einsatz der OH-aciden Verbindungen wird die Biuretbildung beschleunigt und die Bildung von unerwünschten Nebenprodukten herabgesetzt.

Als OH-acide Verbindungen kommen erfindungsgemäß insbesondere Protonensäuren in Betracht. Beispielsweise können eingesetzt werden und haben sich besonders bewährt: Phosphorsäuren und/oder deren Mono- und/oder Dialkyl(aryl)ester und/oder Hydrogensulfate. Verwendung finden vorzugsweise Mono- und/oder Dialkyl(aryl)ester der Phosphorsäure, deren aliphatische, verzweigt aliphatische, araliphatische oder aromatische Reste 1 bis 30, vorzugsweise 4 bis 20, Kohlenstoffatome aufweisen. Beispielsweise kommen Di-(2-ethylhexyl)phosphat und Di-hexadecylphosphat zur Anwendung. Als Hydrogensulfate kommen insbesondere Tetralkylammoniumhydrogensulfate in Betracht, deren aliphatische, verzweigt aliphatische oder araliphatische Reste 1 bis 30, vorzugsweise 4 bis 20, Kohlenstoffatome aufweisen. Insbesondere in Frage kommen Dibutylphosphat und Di-iso-propylphosphat. Bevorzugt wird Di-(2-ethylhexyl)-phosphat. Die beispielsweise in EP-A-259 233 beschriebenen (ar)aliphatischen Carbonsäuren erweisen sich als weniger wirksam.

Die erfindungsgemäß einzusetzenden OH-aciden Verbindungen haben den Vorteil, daß sie schwerflüchtig sind und deshalb, gegebenenfalls als Salze, aus dem Produktgemisch abfiltriert werden können oder als nicht störende Verbindungen im Endprodukt verbleiben oder während der Reaktion ebenfalls nicht störende Zersetzungs-oder Nebenprodukte bilden. Ein weiterer Vorteil ist die gute katalytische Aktivität der Säuren.

Die OH-aciden Verbindungen werden sinnvollerweise in Mengen von 0,01 bis 2 mol-%, vorzugsweise 0,1 bis 0,5 mol-%, bezogen auf das eingesetzte Diisocyanat, eingesetzt.

Um die Bildung von unlöslichen Polyharnstoffen noch besser zu unterdrücken, können zusätzlich Lösungsmittel als Lösungsvermittler verwendet werden. Hierfür geeignet sind beispielsweise Alkoxyalkylcarboxylate und/oder Trialkylphosphate. Erfindungsgemäß bevorzugt eingesetzt werden Methoxypropylacetat, Trimethylphosphat und Triethylphosphat oder beliebige Mischungen dieser Verbindungen.

Die Umsetzung des Diisocyanates mit dem Biuretisierungsmittel wird bei Reaktionstemperaturen von 60 bis 200°C, vorzugsweise bei 100 bis 150°C, durchgeführt.

Die Erfindung wird an nachfolgenden Beispielen erläutert.

### Beispiele 1 bis 5 - erfindungsgemäß (siehe Tabelle 1)

In einem mit Rückflußkühler, Glasrohr mit Fritte und Rührer ausgestatteten Rührkolben wurden 500 g HDI (2,97 mol) vorgelegt. Das Glasrohr mit Fritte war mit einer Apparatur zur Erzeugung von Wasserdampf (ein beheizter Rundkolben mit Tropftrichter, aus dem Wasser langsam in den heißen Kolben dosiert werden kann) und einer Stickstoffleitung verbunden.

Das HDI wurde mit der in der Tabelle angegebenenen Art und Menge an OH-aciden Verbindungen (mol-% bezogen auf HDI) versetzt und unter Durchperlung von Stickstoff auf 130°C vorgeheizt. Danach wurde mit Stickstoff (20 l/h) verdünnter Wasserdampf über die Glasfritte eingeleitet und der Verlauf der Reaktion über die Abnahme des NCO-Gehaltes verfolgt. Bei einem NCO-Gehalt von 40-43 % wurde die Wasserdampfzufuhr (nach ca. 60-75 min) abgestellt und das Reaktionsgemisch für weitere 30 min unter Stickstoff bei 130°C nachgerührt. Danach wurde überschüssiges HDI durch Destillation in einem Dünnschichtverdampfer abgetrennt. Das erhaltene klare und nahezu farblose Sumpfprodukt mit niedrigem Rest-HDI-Gehalt wurde anschließend bei 50°C im Trockenschrank gelagert und der Restmonomerenanstieg nach 6 Wochen gemessen (siehe Tabelle 1).

### Beispiel 6 - Vergleich (siehe Tabelle 1)

Es wurde so wie in den Beispielen 1 bis 5 beschrieben verfahren, jedoch ohne Zusatz von OH-aciden Verbindungen gearbeitet. Die Reaktion war deutlich langsamer und es bildeten sich je nach der zugefahrenen Wasserdampfmenge pro Zeiteinheit mehr oder weniger große Mengen unlöslicher Polyharnstoffe in der Reaktionsmischung (0,01 - 0,5 Gew.-% Polyharnstoff, bezogen auf eingesetztes HDI).

### Beispiele 7 und 8 - erfindungsgemäß (siehe Tabelle 2)

In einem mit Rückflußkühler und Rührer ausgestatteten Rührkolben wurden 500 g HDI (2,97 mol), 250 g Triethylphosphat und die angegebene Art und Menge an OH-aciden Verbindungen (mol-% bezogen auf HDI) vorgelegt und auf 130°C vorgeheizt. Danach wurden unter heftigem Rühren unter einer Stickstoffatmosphäre 6,4 g Wasser zugetropft. Unter heftiger CO₂-Entwicklung reagierte das Gemisch bei 130°C innerhalb von 3 Stunden auf einen NCO-Gehalt von 40-42 % (rückgerechnet auf lösungsmittelfreie Mischung). Danach wurde überschüssiges HDI und Lösungsmittel durch Destillation in einem Dünnschichtverdampfer abgetrennt. Das erhaltene klare und nahezu farblose Sumpfprodukt mit niedrigem Rest-HDI-Gehalt wurde anschließend bei 50°C im Trockenschrank gelagert und der Restmonomeranstieg nach 6 Wochen gemessen.

### Beispiel 9 (Vergleich (siehe Tabelle 2)

Es wurde so wie in den Beispielen 7 und 8 verfahren, jedoch ohne Zusatz von OH-aciden Verbindungen gearbeitet. Die Reaktion war deutlich langsamer und im über der Reaktion befindlichen Gasraum bildeten sich unlösliche Harnstoffe, die sich an nicht beheizten Stellen der Reaktionsapparatur niederschlugen.

**Tabelle 1**

| Herstellung von HDI-Biuret mit Wasserdampf | | | | | |
|---|---|---|---|---|---|
| Vers. | OH-acide Verbindungen [mol-%] | NCO-Gehalt [%] | Rest-HDI-Gehalt [%] | | Produktfarbzahl [Apha] |
| | | | sofort | nach 42 Tagen bei 50°C | |
| 1 | Di-(2-ethylhexyl)-phosphat [0,1] | 23,3 | 0,16 | 0,3 | <10 |
| 2 | Di-(2-ethyhexyl)-phosphat [0,2] | 23,3 | 0,15 | 0,21 | <10 |
| 3 | Di-(2-ethylhexyl)-phosphat [0,4] | 22,9 | 0,24 | 0,26 | <10 |
| 4 | Di-hexadecyl-phosphat [0,2] | 23,1 | 0,19 | 0,3 | <10 |
| 5 | Di-hexadecyl-phosphat [0,46] | 22,8 | 0,12 | 0,29 | <10 |
| 6 | ohne OH-acide Verbindungen | 22,0 | 0,25 | 1,65 | 10-20 |

**Tabelle 2**

| Biuretisierung von HDI mit Wasser in Triethylphosphat | | | | | |
|---|---|---|---|---|---|
| Vers. | OH-acide Verbindungen [mol-%] | NCO-Gehalt [%] | Rest-HDI-Gehalt [%] | | Produktfarbzahl [Apha] |
| | | | sofort | nach 42 Tagen bei 50°C | |
| 7 | Di-(2-ethylhexyl)-phosphat [0,1] | 23,3 | 0,13 | 0,30 | <10 |
| 8 | Di-(2-ethylhexyl)-phosphat [0,2] | 23,3 | 0,18 | 0,29 | <10 |
| 9 | ohne OH-acide Verbindungen | 22,6 | 0,27 | 1,6 | 10-20 |

## Patentansprüche

1. Verfahren zur Herstellung von Biuretgruppen enthaltenden Polyisocyanaten durch katalytische Umsetzung von aliphatischen und/oder cycloaliphatischen Diisocyanaten mit einem Biuretisierungsmittel, dadurch gekennzeichnet, daß als Biuretisierungsmittel Wasser in feindisperser Form eingesetzt wird und die Reaktion im Beisein von OH-aciden Verbindungen, ausgenommen Carbonsäuren, erfolgt.

2. Verfahren nach Anspruch 1, dadurch gekennzeichnet, daß als Biuretisierungsmittel Wasserdampf eingesetzt wird.

3. Verfahren nach Anspruch 1 oder 2, dadurch gekennzeichnet, daß als Biuretisierungsmittel Wasserdampf in Verbindung mit einem Inertgas eingesetzt wird.

4. Verfahren nach einem der Ansprüche 1 bis 3, dadurch gekennzeichnet, daß als OH-acide Verbindungen Phosphorsäuren und/oder deren Mono- und/oder Dialkyl(aryl)ester eingesetzt werden.

5. Verfahren nach einem der Ansprüche 1 bis 3, dadurch gekennzeichnet, daß als OH-acide Verbindungen Hydrogensulfate eingesetzt werden.

6. Verfahren nach Anspruch 5, dadurch gekennzeichnet, daß als OH-acide Verbindungen Tetraalkylammoniumhydrogensulfate eingesetzt werden.

7. Verfahren nach einem der Ansprüche 1 bis 6, dadurch gekennzeichnet, daß zusätzlich Methoxypropylacetat und/oder Trimethylphosphat und/oder Triethylphosphat als Lösungsvermittler eingesetzt werden.

8. Verfahren nach einem der Ansprüche 1 bis 7, dadurch gekennzeichnet, daß als Diisocyanat Hexamethylendiisocyanat verwendet wird.

9. Verfahren nach einem der Ansprüche 1 bis 7, dadurch gekennzeichnet, daß als Diisocyanat Isophorondiisocyanat eingesetzt wird.

## Claims

1. A process for the preparation of biuret-containing polyisocyanates by catalytic reaction of aliphatic and/or cycloaliphatic diisocyanates with a biuretizing agent, which comprises employing water in finely disperse form as biuretizing agent and carrying out the reaction in the presence of OH-acidic compounds, excluding carboxylic acids.

2. A process as claimed in claim 1, wherein the biuretizing agent employed is steam.

3. A process as claimed in claim 1 or 2, wherein the biuretizing agent employed is steam in conjunction with an inert gas.

4. A process as claimed in any one of claims 1 to 3, wherein the OH-acidic compounds employed are phosphoric acids and/or the mono- and/or dialkyl(aryl) esters thereof.

5. A process as claimed in any one of claims 1 to 3, wherein the OH-acidic compounds employed are hydrogen sulfates.

6. A process as claimed in claim 5, wherein the OH-acidic compounds employed are tetraalkylammonium hydrogen sulfates.

7. A process as claimed in any one of claims 1 to 6, wherein, in addition, methoxypropyl acetate and/or trimethyl phosphate and/or triethyl phosphate are employed as solubilizers.

8. A process as claimed in any one of claims 1 to 7, wherein the diisocyanate used is hexamethylene diisocyanate.

9. A process as claimed in any one of claims 1 to 7, wherein the diisocyanate employed is isophorone diisocyanate.

## Revendications

1. Procédé pour la préparation de polyisocyanates contenant des groupes biuret, par mise en réaction catalytique de diisocyanates aliphatiques et/ou cycloaliphatiques avec un agent de biurétisation, caractérisé en ce qu'on met en oeuvre, à titre d'agent de biurétisation, de l'eau sous forme finement dispersée et la réaction a lieu en présence de composés acides contenant un ou plusieurs groupes OH, à l'exclusion d'acides carboxyliques.

2. Procédé selon la revendication 1, caractérisé en ce qu'on met en oeuvre de la vapeur d'eau à titre d'agent de biurétisation.

3. Procédé selon la revendication 1 ou 2, caractérisé en ce qu'on met en oeuvre, à titre d'agent de biurétisation, de la vapeur d'eau en liaison avec un gaz inerte.

4. Procédé selon l'une quelconque des revendications 1 à 3, caractérisé en ce qu'on met en oeuvre, à titre de composés acides contenant un ou plusieurs groupes OH, des acides phosphoriques et/ou leurs esters mono- et/ou dialkyl(aryliques).

5. Procédé selon l'une quelconque des revendications 1 à 3, caractérisé en ce qu'on met en oeuvre, à titre de composés acides contenant un ou plusieurs groupes OH, des hydrogénosulfates.

6. Procédé selon la revendication 5, caractérisé en ce qu'on met en oeuvre, à titre de composés acides contenant un ou plusieurs groupes OH, des hydrogénosulfates de tétraalkylammonium.

7. Procédé selon l'une quelconque des revendications 1 à 6, caractérisé en ce qu'on met en outre en oeuvre le méthoxypropylacétate et/ou le triméthylphosphate et/ou le triéthylphosphate à titre d'agent de solubilisation.

8. Procédé selon l'une quelconque des revendications 1 à 7, caractérisé en ce qu'on utilise, à titre de diisocyanate, l'hexaméthylènediisocyanate.

9. Procédé selon l'une quelconque des revendications 1 à 7, caractérisé en ce qu'on met en oeuvre, à titre de diisocyanate, l'isophoronediisocyanate.
